# EUROPEAN PATENT SPECIFICATION

(11) **EP 2 760 544 B1**
(45) Date of publication and mention of the grant of the patent: **06.04.2022**
(21) Application number: 12837229.9
(22) Date of filing: 01.10.2012
(51) Int. Cl.: G06Q 50/22, A61N 5/10

(54) **SEMANTIC RADIATION TREATMENT PLAN OPTIMIZATION GUIDANCE**
SEMANTISCHE ANLEITUNG ZUR OPTIMIERUNG EINES BESTRAHLUNGSBEHANDLUNGSPLANS
CONSEILS D'OPTIMISATION DE PLAN DE TRAITEMENT RADIOLOGIQUE SÉMANTIQUE

(30) Priority: 30.09.2011 US 201113249669
(43) Date of publication of application: 06.08.2014
(73) Proprietor: Varian Medical Systems International AG, 6312 Steinhausen (CH)
(72) Inventor: TOIMELA, Lasse, 02740 Espoo (FI); NORD, Janne, 02620 Espoo (FI); ZANKOWSKI, Corey, San Jose, CA 95124 (US)
(74) Representative: Foster, Mark Charles
(86) International application number: PCT/US2012/058355
(87) International publication number: WO 2013/049845

(56) References cited:
- WO-A2-2005/035061
- WO-A2-2007/002642
- WO-A2-2009/153057
- WO-A2-2010/018477
- US-A1- 2007 071 168
- US-A1- 2008 152 085
- US-A1- 2010 232 572
- US-A1- 2011 153 547

## Description

### TECHNICAL FIELD

The present disclosure relates generally to the field of radiation therapy and more specifically to the field of radiation therapy treatment plan development and optimization.

### BACKGROUND

Oncology and Radiotherapy technology continues to evolve, accelerating the expansion of the envelope of possible treatments and best practices associated with those possible treatments. At the same time, the complexity of clinical decisions is increasing non-linearly, resulting in a rapidly widening gap between actual practice and best practices, especially in emerging markets.

For example, when medical imaging is necessary in the course of radiation therapy, several systems may be used, such as X-ray, magnetic resonance imaging (MRI), computed tomography (CT), and others. When CT or MRI imagery, for example, is used, a series of two-dimensional images are taken from a three-dimensional volume. Here, each two-dimensional image is an image of a cross-sectional "slice" of the three-dimensional volume. The resulting collection of two-dimensional cross-sectional slices can be combined to create a three dimensional image or reconstruction of the patient's anatomy. This resulting three-dimensional image or three-dimensional reconstruction will contain organs of interest. Those organs of interest include the organ targeted for radiation therapy, as well as other organs that may be at risk of radiation therapy exposure. The portion of the three-dimensional image or reconstruction that contains the organs of interest may be referred to as structures of interest or volumes of interest.

These one or more structures of interest may be viewed in several ways. A first and simplest way to view the structure(s) of interest would be to merely view the original CT or MRI image slices for the patient, with each slice containing a view of the structure(s) of interest. A second, and more complicated method to view the structure(s) of interest would be to combine the series of two-dimensional cross-sectional slices into a single three-dimensional representation where the structure(s) of interest may be represented as solid, opaque, or translucent, etc., objects that may then be manipulated (e.g., rotated) to allow viewing from multiple angles.

One purpose of the three-dimensional reconstruction of the structure(s) of interest containing diseased or abnormal tissues or organs is the preparation of a three-dimensional radiation therapy treatment plan. Radiation therapy treatment plans are used during medical procedures that selectively expose precise areas of the body, such as cancerous tumors, to specific doses of radiation to destroy the undesirable tissues. To develop a patient-specific radiation therapy treatment plan, information is extracted from the three-dimensional model to determine perimeters such as organ shape, organ volume, tumor shape, tumor location in the organ, and the position or orientation of several other structures of interest as they relate to the affected organ and any tumor.

Such radiation therapy treatment plans can make use of different optimization processes than can be used to achieve an improved treatment plan. Optimization may consist of computationally finding an optimal flow of particles to fulfill user (e.g. clinician) given radiation tolerance limits or for example, finding an optimal radiation field directionality and count. Typically, this is an iterative trial and error process and can be time consuming. Examples of treatment plan optimisation processes are described in publications WO 2007/002642 A2 and WO 2010/018477 A2.

As evolving technologies provide increasingly complicated radiation therapy treatment planning possibilities, the gap between actual clinical practice in treatment planning and possible best practices in treatment planning increases. Therefore improved methods for realizing and communicating improved results using emerging technological innovations are required.

### SUMMARY OF THE INVENTION

This present invention as defined by the claims provides a solution to the challenges inherent in achieving radiation therapy treatment planning best practices through improved optimization. In a method according to one embodiment of the present invention, a method for improving treatment plan optimization is disclosed. The method comprises generating a first radiation therapy treatment plan for a patient using a selected treatment type from a database and optimizing the first radiation therapy treatment plan. A selection of metrics from the first radiation therapy treatment plan are identified and compared to metrics from aggregated prior patient records selected from the database. At least one objective and its associated parameters are identified that affects the ability of the first radiation therapy treatment plan to meet an identified metric. The parameters of the objective that affects the identified metric are compared to competing parameters of another objective. At least one alternative treatment step for the treatment plan is determined that will improve the identified metric. The identified metric is improved by adjusting the parameters of the identified objective at the expense of the competing parameters and their associated objective.

### BRIEF DESCRIPTION OF THE DRAWINGS

The present invention will be better understood from a reading of the following detailed description, taken in conjunction with the accompanying drawing figures in which like reference characters designate like elements and in which:
Figure 1 illustrates an exemplary simplified block diagram illustrating an embodiment of a knowledge-based treatment planning system, in accordance with an embodiment of the present disclosure;
Figure 2 illustrates an exemplary simplified block diagram illustrating an embodiment of a knowledge-based treatment planning system with improved Optimizer, in accordance with an embodiment of the present disclosure;
Figure 3 illustrates steps of an exemplary process for optimizing a radiation therapy treatment plan, in accordance with an embodiment of the present disclosure; and
Figure 4 illustrates steps of an exemplary process for optimizing a radiation therapy treatment plan, in accordance with an embodiment of the present disclosure.

### DETAILED DESCRIPTION

### NOTATION AND NOMENCLATURE:

Some portions of the detailed descriptions, which follow, are presented in terms of procedures, steps, logic blocks, processing, and other symbolic representations of operations on data bits within a computer memory. These descriptions and representations are the means used by those skilled in the data processing arts to most effectively convey the substance of their work to others skilled in the art. A procedure, computer executed step, logic block, process, etc., is here, and generally, conceived to be a self-consistent sequence of steps or instructions leading to a desired result. The steps are those requiring physical manipulations of physical quantities. Usually, though not necessarily, these quantities take the form of electrical or magnetic signals capable of being stored, transferred, combined, compared, and otherwise manipulated in a computer system. It has proven convenient at times, principally for reasons of common usage, to refer to these signals as bits, values, elements, symbols, characters, terms, numbers, or the like.

It should be borne in mind, however, that all of these and similar terms are to be associated with the appropriate physical quantities and are merely convenient labels applied to these quantities. Unless specifically stated otherwise as apparent from the following discussions, it is appreciated that throughout the present invention, discussions utilizing terms such as "processing" or "accessing" or " executing" or "storing" or "rendering" or the like, refer to the action and processes of a computer system, or similar electronic computing device, that manipulates and transforms data represented as physical (electronic) quantities within the computer system's registers and memories and other computer readable media into other data similarly represented as physical quantities within the computer system memories or registers or other such information storage, transmission or display devices. When a component appears in several embodiments, the use of the same reference numeral signifies that the component is the same component as illustrated in the original embodiment.

This present invention provides a solution to the increasing challenges inherent in improving radiation therapy best practices. Various embodiments of the present disclosure provide the use of a knowledge base incorporating a mixture of patient records and statistical models to simplify and improve the optimization of a radiation therapy treatment plan. As discussed in detail below, the optimization methods map a clinician's possible clinical intentions into optimization parameters based on data analysis and a clinical knowledge base, such that the clinical decisions made are mapped and semantic expressions in human understandable language are provided to the clinician to describe other options (e.g. alternative treatment options involving parameter setting trade-offs) to improve outcomes based on the knowledge base statistics. Such alternative options may be necessary when a clinician's clinical intentions are conflicting with each other, or with "conventional wisdom" or other treatment plans found in the knowledge base. Further, the actions of the clinician in response to the provided treatment alternatives can also be captured in human understandable language. Such captured clinical actions can then be used later as additional options for future treatment plan optimizations. Exemplary embodiments can map parameters that drive algorithms that are often unseen by the clinician/user and map those parameters to a user understandable objective. In other words, rather than requiring a clinician to specify, for example a particular dose weight, the clinician can instead ask for a target homogeneity.

### Initial Treatment Plan Development:

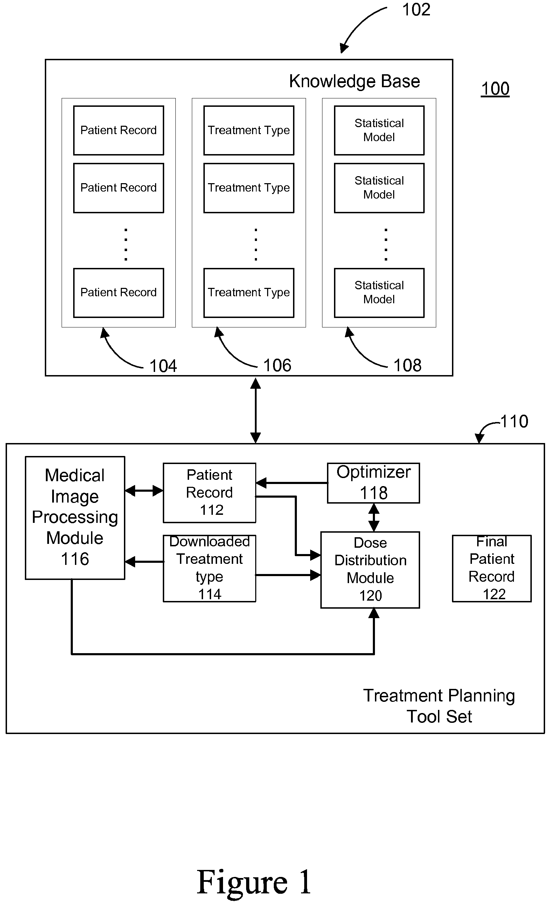

Figure 1 illustrates an embodiment utilizing a knowledge base incorporating a mixture of patient records and statistical models to simplify the generation of optimized radiation therapy dose distribution treatment plans. Individual patient records can include all of the medical records/documents for a particular previous patient or hypothetical patient, such as treatment plan utilized, treatment outcomes, treatment type, metadata, and medical imaging such as CT and MRI slides. An exemplary radiation therapy treatment planning system 100, as illustrated in Figure 1, comprises a knowledge base 102 and a treatment planning tool set 110. The knowledge base 102 of Figure 1 comprises a plurality of patient records 104, a plurality of treatment types 106, and a plurality of statistical models 108. The treatment planning tool set 110 of Figure 1 comprises a current patient record 112 (e.g., complete treatment plan), a downloaded treatment type 114, a medical image processing module 116, an optimizer 118, a dose distribution module 120, and a completed patient record 120 (e.g., comprising a final, approved treatment plan). The treatment planning tool set searches through the knowledge base for prior patient records that are similar to the current patient case, and using current patient information, a selected treatment type 106, and selected statistical models 108, generates an optimized treatment plan in the patient record 112 that is then approved by the clinician.

The knowledge base 102 allows the development of radiation therapy treatment plans and their dose distributions based on prior clinical experience. By using statistical models 108 based on prior treatment plans 104 approved by the clinician, as well as selecting a most commonly used treatment type 114, based on the current patient's meta data, embodiments are able to create treatment plans that can then be optimized by an optimization system 118, and then refined by the clinician. The development and optimization of a treatment plan is also discussed in patent publication US 8774358 , titled "RADIATION THERAPY TREATMENT PLAN IMPROVEMENT THROUGH USE OF KNOWLEDGE BASE," by Zankowski.

Typically, an optimization system 118 consists of both clinically relevant restraints, such as radiation tolerance limits or priorities of organs at risk and irradiation targets such as tumors. Additionally, a plurality of other parameters can also be used to guide the optimization system 118, such as how many iterations at most an optimizer 118 will perform, at what granularity the optimizer 118 analyzes the problem (for example, how accurately the optimizer calculates an estimate of absorbed dose to a patient), and what treatment device parameters should be optimized, etc.

Typically, the final result achieved by the optimization system 118 is a compromise between several competing objectives and is limited by or affected by the associated parameters given. A clinician/user must decide how to adjust these objectives and their parameters to achieve the best possible treatment plan for each patient. As noted above, this is typically an iterative trial and error process and can be time consuming.

After the optimization has begun, the optimization system 118 has a large amount of information about the current optimization state available compared to the information shown to the user/clinician. A significant portion of this information can be complex in nature and with multiple dependences among different objectives can also be especially difficult to understand. Such a situation results in a time consuming, iterative process attempting to optimize the treatment plan.

As discussed by Zankowski, the knowledge base can be searched for a combination of optimization objectives that can be applied by an optimizer to optimize the dose distribution. For example, an average organ at risk dose volume histogram, a mean cohort organ at risk dose volume histogram and average organ at risk objectives are selected from the knowledge base. In one embodiment, there are a plurality of organs at risk with associated average organ at risk dose volume histograms and cohort average organ at risk dose volume histograms. In other words, the optimizer will have an objective of at least meeting the average dose distribution achieved previously in similar patients by the clinician. The optimizer may make use of 2D penalty maps to help shape the dose distribution such that doses received by one or more critical organs at risk are minimized and the dose to the target organ is maximized. Other objective functions may include biological outcome maps and a dose distance to target histogram. Using the objectives and field arrangement, the optimizer can provide an optimal 3D dose distribution, fluences and associated dose volume histograms for the current patient. Optimally, these results will fall within the historically accepted range for a patient with a similar disease type and treatment type.

In further steps, the clinician can review the results of the optimization and adjust optimization parameters. By changing the objectives, the clinician can drive the optimizer to a new result. The new objectives are appended into the patient organ at risk objective functions. The resulting new dose distribution, fluence and dose volume histogram are also stored in the patient record. Once the clinician is satisfied with the final result and having verified that all treatment plan elements are consistent, a dose distance to target histogram is calculated for the current patient. Finally, the knowledge base can be updated with the current patient information. After determining which population cohort the current patient belongs in, the field geometry is updated for the selected cohort, while population statistics and the cohort population statistics are also updated with the new data.

Therefore, as described by Zankowski, the clinician may select conservative objective values (which are more difficult for the optimizer to solve) for the critical organs at risk and for less critical organs or areas in the desired distribution map that are not very well specified (e.g., areas outside the target organ and outside the critical organs at risk), a more relaxed dose limit can be selected. Such a combination of conservative dose objectives and relaxed dose objectives prevent the optimizer from unnecessarily reducing the dose levels.

In an exemplary embodiment, the optimizer 118 begins with a *desired* dose distribution and generates a set of fluences (Fl) and resulting 3D dose distribution (D3D) that will as closely as possible match that desired dose distribution. While the desired dose distribution may have areas that are not physically achievable, the optimizer can smooth them out and provide the clinician a reasonable dose distribution that is achievable. This reasonable 3D dose distribution (D3D) provides a starting point for further optimization and adjustment by the clinician.

Zankowski illustrates that the RT structures (RTS), the distance to target map (D2T), and treatment technique 114 in the current patient record 112 can be used to select an average organ at risk dose volume histogram (OAR DVH), a mean cohort organ at risk dose volume histogram (OAR DVH) and average organ at risk objectives (OAR Objectives), respectively, from the knowledge base 102 to be fed into the optimizer 118 for each of the one or more organs at risk. By taking an average of the organ at risk dose volume histograms (OAR DVH) for the population and selected cohort population, the optimizer 118 may use an objective or goal that is based upon an average of the results achieved by the clinician while treating previous patients. In another embodiment, additional objectives may also be selected, such as biological models and a dose distance to target histogram. A biological model can estimate or predict the biological outcome that may result from a given dose distribution. The above objectives are combined to create a patient-specific organ at risk objective function (OAR) that is stored in the patient record 112. The organ at risk objective function (OAR) provides a goal for the optimizer 118 to reach for. As discussed above, based on the supplied organ at risk objective function (OAR), the optimizer 118 produces a set of fluences (Fl) and a 3D dose distribution (D3D) that comes closest to meeting those assigned objectives for the target organ and the one or more organs at risk.

### Semantic Radiation Treatment Plan Optimization Guidance:

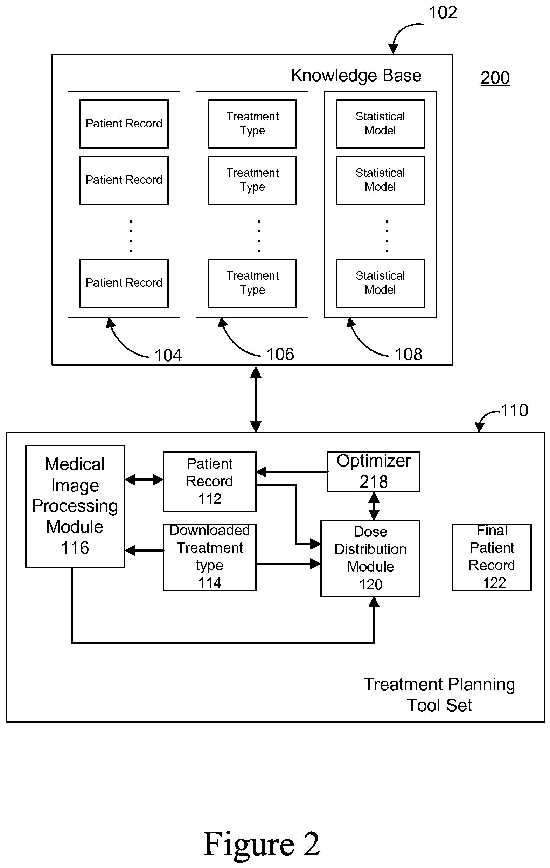

Figure 2 illustrates an improved Optimizer 218. The remaining modules and blocks illustrated in Figure 2 are the same as in Figure 1 and are so annotated. As illustrated in Figure 2, an exemplary embodiment includes an optimizer 218, as discussed below, that analyzes the data it has available. Based on this analysis, the optimizer 218 can notify the clinician/user that the optimizer 218 has now found a way to further improve the treatment plan and offers optional treatment alternatives to achieve improved clinical intents the user/clinician might have. The treatment plan alternatives offered are based upon an analysis of the trade-offs possible between competing objectives and their parameters, such that a given metric can be improved by improving one set of parameters at the expense of another set of parameters. The following table, Table 1, provides an exemplary list of possible alternatives with a user selectable option or guiding textual information.

**Table 1**

| | |
|---|---|
| Optional Improvement that may be selected by the Clinician/User: | Actual Steps Taken by the Optimizer that Resulted in a User Selectable Option or Guiding Textual Information: |
| To improve target dose homogeneity: increase the priority of the target structure and bring the upper and lower dose constraints closer to the prescription dose. | The optimization system has internally tried to improve the target dose homogeneity and seen it does not immediately worsen the objectives of other organs. |
| 5% of the rectum volume receives more than 68Gy, which is above the ICRU recommendations. If the clinician wishes to bring the dose down, an additional constraint can be inserted with 5% at 68GY with priority "high." | The optimization system has compared the dose volume histograms of critical organs against ICRU recommendations. |
| The clinician has requested the total number of treatment fields to be four. By the clinician inserting one more field coming from gantry angle 128 degrees with a couch rotation of 0 degrees, a better critical organ sparing could be achieved. | The optimization system has tested the possible achievable plan quality with a number of requested fields and seen a significant improvement in plan quality with the additional of one more field. |
| The structures "PTV" and "left lung" are overlapping. The clinician can either: generate a separate structure for the overlapping volume and assign a differing constraint to achieve a better target coverage, or the clinician can exclude the overlapping part of the critical organ from optimization. | The optimization system has done geometrical analysis of structures with constraints. It is known a priori that the overlapping volumes with competing constraints can limit the whole structure objective. |
| A small region of high dose was generated outside the target. The clinician can generate an additional guidance structure for this region and assign a limiting constraint that reduces the hot region outside the target. | The optimization system has analyzed the dose inside the patient also in areas where no user delineated targets exists and found a possibly significant deviation from average dose to body. |
| The rectum dose could be further reduced without sacrificing target dose coverage, by the clinician optionally bringing the rectum constraints to a lowest non-compromising level. | The optimization system has tested lowering the dose to a known critical organ. The dose could be lowered somewhat without compromising the target dose coverage. |
| The clinician is notified of the situation and offered a choice of which of the competing objectives could be traded for the improved target dose homogeneity. For example, "target dose homogeneity does not meet the limits you have defined. Would you like to a) improve the target by decreasing the importance of lung V20 constraint b) improve the target by removing constraints from the lung overlapping with the target, or c) accepting the target dose homogeneity and do nothing. " | The optimization system has identified that target dose homogeneity is bad and identified competing objectives that restrict target dose homogeneity improvements. |

Such an optimizer 218 allows a clinician/user to see their clinical observations and intents mapped during and after optimization into a set of operations to be performed in the optimization parameter space and can even allow the Optimizer 218 to automatically find the right value. Such an improvement possibility (the generation of treatment alternatives) can be automatically detected by the Optimizer 218 or requested by the clinician/user. For example, the clinician could evaluate that target dose homogeneity needs to be improved and request improving the target dose homogeneity objectives. The Optimizer 218 could find any competing objectives that relate to homogeneity and optionally evaluate how big a change they would make. Then the Optimizer 218 could offer a list of alternatives on how to improve the selected objective (in this example homogeneity). For example, critical organ objectives 1 and 2 compete with target dose homogeneity, but critical organ objective 3 does not. Based on the analysis the clinical/user is given an "alternative" to modify the critical organ objective 1 or 2, and optionally, the clinician/user is also shown the effect of the alternatives on the treatment plan. These alternatives are provided in clinically meaningful, human-understandable language.

With prior patient records and statistical models in a knowledge base 102, the Optimizer 218 has the ability to mine the "data" found in the knowledge base 102 and generate statistics, or analyze different metrics and look for distributions in those metrics. Therefore, an exemplary Optimizer 218 can provide different statistical descriptions with the prior knowledge available in the treatment plans from prior patient records. The clinician/user and the Optimizer 218 itself can start to learn from looking at the data in the knowledge base 102: what's the usually achievable goal and what is not. Or stated another way: what's unusually achievable versus what's unusually missed in terms of the goals.

The Optimizer 218 can replace multiple complex parameters with one single, simple statement. For example, the Optimizer 218 enables the clinician/user to achieve a variety of different goals by reducing multiple complex parameters down into a single simple statement for the user, such as: I want to improve target dose distribution by increasing a target dose and increasing priority against some competing constraint. Such a semantic statement describes a competing constraint against the target dose and describes by how much the target dose will be improved by reducing the competing parameter.

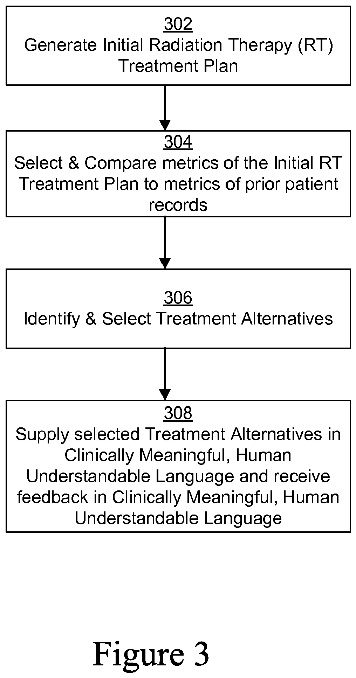

In one exemplary process, as illustrated in Figure 3, in step 302, an initial radiation therapy treatment plan is generated. The optimization system 218 can utilize the initial parameters set by the clinician, and based on those parameters and statistical models in the knowledge base can produce the initial treatment plan result. Then, in step 304, a selection of metrics for the initial radiation therapy treatment plan are compared to metrics of an aggregate of selected prior patient records. Prior patient records can be selected according to a variety of qualifying criteria, such as prior patient records with similar metadata and desired outcomes. As discussed in detail below, the selected metrics for the initial radiation therapy treatment plan can be provided percentile scores. In step 306, based on previous successful treatment plans (by the clinician or by others that the clinician is interested in and/or able to access) and the statistical models in the knowledge base, the Optimizer 218 can provide a list of treatment alternatives such than when accepted by the clinician, the stated changes would produce the stated result. In other words, a particular change to the parameters for an objective will result in an improved metric percentile score at the expense of a competing objective. In step 308, the selected treatment alternatives are provided in clinically meaningful, human-understandable language. Such human understandable questions allow the clinician to compare predicted metrics with an aggregate of prior metrics they have achieved so far, and based on their own clinical decisions, the clinician will select the best optimization for their current patient based on a desire for them to receive a particular outcome. For example, the clinician can choose from several trade-offs among competing objectives and their corresponding parameters to improve the target dose while maintaining a desired organ at risk exposure below a maximum value. Lastly, when a clinician chooses not to select a treatment alternative, feedback in clinically meaningful, human understandable language is also possible.

### Selecting and Comparing Metrics:

In another exemplary embodiment, the Optimizer 218 looks at each organ at risk separately, each target separately, and analyzes the homogeneity (dose distribution) versus individual target and organ at risk exposures. Such an embodiment breaks up the problem into different metrics to analyze the current treatment plan results for a plurality of physical organs or features. Examples include: how well the treatment plan performs at the rectum, how well it performs at the prostate, how well it performs in sparing the femoral heads, and how well it performs at minimizing high dose spots outside the target area. Such an analysis uses the statistical models in the knowledge base to compare the current treatment plan's predicted results to a statistical patient. By looking at individual "components" of the treatment plan, the optimizer 218 enables a clinician to look at these different metrics at a human understandable level and "lock down" a selection of highest priority metrics or parameters (or individual components that are weighed higher than other components). With a selection of metrics or parameters locked down, the Optimizer 218 is then able to adjust the remaining parameters so that the Optimizer 218 can determine if the current treatment plan can be further improved by adjusting certain of the remaining parameters. Such adjustments can be performed in tiers, with a first tier of parameters locked down, followed by a second tier of parameters locked down, and finally a third tier of parameters locked down.

As the Optimizer 218 determines what the critical metrics are, the treatment plan can be driven to achieve a desired good outcome. In one embodiment, to accomplish this desired good outcome, the statistical analysis performed by the Optimizer 218 can start to learn what can be referred to as "implicit rules." Such implicit rules, could for example, be learned in an overlap region situation: is it the organ at risk that will take precedence when the overlap region is prostate/rectum, or is it the target organ that takes precedence in that case, in terms of what the final result may be. By looking at the final results when similar trade-offs were made in previous similar treatment plans found in the knowledge base, the Optimizer 218 can select or suggest a particular tradeoff for the current treatment plan, based on the results from the previous cases. Such decisions can be built into the rule set that the Optimizer 218 can follow.

### Comparing Radiation Therapy Treatment Plan Metrics to Prior Results:

In one embodiment, feedback from the Optimizer 218 can show the clinician how well they are doing versus an aggregate of prior treatment plans with similar metadata, focusing on a select number of key attributes or metrics. Once 3-5 key attributes or metrics of an optimal treatment plan are identified that would result in a treatment plan that meets desired objectives, the Optimizer 218 can provide analysis, showing the clinician/user that they are, for example, in the 63th percentile for one metric, but in the 20th percentile in another metric, or in the 90th percentile for an additional metric. Such percentile evaluations show how the current predicted results compare to those aggregated results achieved by past successful treatment plans. In other words, a 60th percentile result for a metric tells the clinician that their selection of parameters for any objectives that effect the above metric are as good as or better than 60% of the results achieved by the prior treatment plans considered. So if the results are for example, 63th, 20th, and 90th percentile, there may be a tradeoff available, for example, the clinician could relax the 90th percentile metric result to improve the 20th percentile metric result. However, there could also be factors that affect the decision: an attribute or metric that is more important than the dose distribution that drove the result.

In another embodiment, the Optimizer 218 also considers how the parameters of objectives for different metrics are inter-related. Generally, objectives and their associated parameters are not independent: if one parameter is changed to produce a desired objective or metric result, another parameter and its corresponding objective or metric could also be affected. Therefore, when one or more parameters are changed, the combined effect of the change to any related inter-dependent parameters will produce a result that is a result of the individual changes to each of the dependent parameters based on the change to one of the parameters. The Optimizer 218 will have identified those objectives and their parameters that are considered important and could be affected by changes to parameters for other objectives.

In exemplary embodiments, such an optimization system can begin moving treatment planning out of the realm of only human beings and also into the realm of automation, where clinicians can establish what an acceptable result is, based on selected attributes or metrics. In other words, clinicians can drive the attributes or metrics that they desire and define the clinician objectives, rather than merely selecting numerical parameter settings and dose volume histogram constraints, in a series of iterations, while attempting to find an optimal treatment plan result. By using the statistical data available in the knowledge base which contains both previous treatment plans as well as statistical models, a norm, or a normal result, based on past knowledge and the current patient attributes can be determined. By defining bad results from good results (e.g. more dose to tumor, less dose to critical organs at risk), the optimization system can drive a separation between the dose to target result versus the dose to organs at risk.

### Developing Treatment Alternatives:

In one embodiment, the optimization system attempts to raise the bar of acceptable, possible results and outcomes automatically and continually. Such improvements can be made by locating statistical aberrations in the knowledge base. Sometimes an aberration will put a clinician into a position where they know a treatment plan result could be much better than what the system is providing. In other words, the knowledge base needs to be driven to a better result. Sometimes an aberration will be on the other side: a clinician will have a result that is far better than what the statistical norm is. A statistical analysis of the previous results stored in the knowledge base can be used to determine why the aberration is so much better than the norm. By learning what resulted in this aberration, such changes can be incorporated into the knowledge base to improve the expected results.

In one embodiment, the optimization system can take the initial parameters provided by the clinician and compare the projected optimized treatment planning results with the statistical results found in the knowledge base and provide a selection of objective alternatives that the clinician could select to improve the current treatment plan result. For example, the optimization system can suggest the addition of a field, a change of angle, or changes in input levels along with stated expected results from incorporating the suggested alternatives. Such an embodiment could be used to determine why a clinician is short of an average result when compared to a particular statistical average, such as the statistical results achieved by a particular clinical group.

In other words, there could be different classes of solutions based on different groups of expert clinicians. It could be that at an individual patient level, one solution is better than another. Some patients may do better under one method rather than under another method. If the system noted that one particular clinical group always seemed to have better outcomes for a patient with a particular set of metadata than any other clinical group, the optimization system could suggest the use of that particular clinical group's treatment plan and any associated treatment type instead of another group's treatment plan for a patient with particular metadata.

The system can also be used to provide a comparison between what the system would produce if the clinician used one setup or treatment method (e.g., treatment type), and what would happen if the clinician used another known good treatment plan setup or method (e.g., treatment type). As soon as a clinician approves a change to a different clinical group, it could be an external validation that the external human mapping comparing one case to another should be followed. There are many different reasons for a clinician using a particular treatment method. Sometimes one treatment method or treatment type will produce improved results over another treatment method or treatment type. But there may be occasions when a treatment method will be selected when another treatment method has been shown to result in improved results. Such reasons can include a complex and inter-related variety of reasons, such as for example: economic considerations, political decisions, specific patient concerns, medical necessity, logistical requirements, and risk of complications, etc. In other embodiments, there can be a selection between treatment method tradeoffs, such as while a particular treatment type has a shorter treatment time, it is also harder to monitor.

Embodiments of an exemplary optimization system can map the decision points of a clinician's decisions when making, for example, a trade-off between extending treatment times by a factor of three and gaining a more precise treatment with higher percentile metric results achieved for the treatment plan. Or the clinician could have opened the margins and treated the patient more quickly, but accepted a lower percentile achievement. Such could be the choice when treating a patient that cannot tolerate a longer treatment time. These selections between trade-offs are mental decisions that a clinician would make. Embodiments of the exemplary optimization system looks to the knowledge base to understand why the clinician made their decision by mapping the metrics selected.

In another embodiment, the treatment plan outcome can be predicted to require 25 minutes, for example. If the clinician knows that a treatment is going to take 25 minutes, then the patient will be scheduled for a 25 minute slot and not a 15 minute slot or a 45 minute slot. Such analysis would result in improvements in efficiency and productivity.

### Feedback on Treatment Alternatives:

In one embodiment, when the clinician intervenes and overrules what the knowledge base's statistical models would have incorporated into the treatment plan, the system is able to capture a reason for the deviation in clinically meaningful, human understandable language. Such interactive feedback will allow the clinician to report why they changed the treatment plan from the suggested treatment plan, such that that decision can help drive the knowledge base to provide an improved treatment plan in the future. Such improvements can be tracked by the system and incorporated in future editions of the system.

If the actions that a clinician takes are already in the "mapping," and is already in the desired human readable form, then it's a self-implemented process. When the clinician is making decisions about how to improve the outcome, there would be human readable decisions that were made that resulted in the final product. In other words, there are certain actions where certain predicted results were selected (e.g. bring down the dose to the organ at risk, or a shorter time requirement in exchange for a different dose distribution). Such an embodiment would provide a detailed description of the decisions that were made by the clinician and what the outcome was. There may also be other actions and decisions that haven't yet been mapped and need to be captured.

If there was a primary reason why a certain decision was made (such that it went against what the knowledge base recommended), the clinician could keyboard in a reason for why the decision was made. These decisions entered in human understandable language could provide a way for the system to have feedback for why the clinician deviated from the system's recommended treatment plan. Such implementations could map the clinician's goals into the knowledge base, while also providing a mechanism for capturing human decision-making that is outside of the system current semantics, that in future iterations can be added to the semantic language of treatment plan optimization. To improve the amount of time for inputting feedback, there can also be an interface for clinicians to choose a few common reasons and a space for inputting a detailed message if the clinician so desired.

In another exemplary embodiment, there can be a feedback box that the clinician can use to explain why they deviated from the determined best treatment plan based upon the current patient's attributes and the aggregated results from the knowledge base. It allows the clinician to document why the change was made. Later, another clinician can then be provided with an alternative that notes that while the current treatment plan appears to be the best according to the knowledge base, there was a clinician that chose to make a stated change for a stated reason. Therefore, if there is a deviation from the recommended treatment plan as provided by the knowledge base, there can be an explanation for such deviation that can be presented in human readable form that could later be incorporated into the system to improve future outcomes by providing that deviation as a selectable human-understandable decision. Such an implementation can be useful in for example medical trials, where a pre-regimented treatment approach would have to be followed if a clinician wished to be in the medical trial. Under such an implementation, the clinician would have to follow the prescribed procedure precisely, and any deviation from that prescribed procedure would be documented with a justification, or the system may not allow the deviation. In other words, the system implemented for such a clinical trial wanted the clinicians to be required to state why they were deviating if they chose not to follow the clinical trial

In another embodiment, the system can provide a particular result that it considers a best solution for a particular patient, based on the initial parameters selected by the clinician and an aggregate of previous treatment plans from the knowledge base along with statistical models. Once a "best solution" has been determined by the optimizer 218, an interactive mode can be offered to the clinician. In such an interactive mode, the clinician is provided with a series of percentile scores (or a toggle bar be provided for parameters that can be interfaced in such way) for a selection of attributes or metrics that define how well the current optimized treatment plan compares to the aggregated previous patient records. The clinician will then be offered a selection of possible treatment alternatives that provide treatment trade-offs between competing objectives that effect the identified attributes or metrics. The clinician could then accept one or more of these recommended treatment alternatives. As discussed above, these suggested alternatives can be based on changes to the available parameters for the objectives that affect the attribute or metric of interest as well as the parameters for competing objectives. Further those identified parameters are be further defined as to whether or not they are considered primary parameters, such that when other parameters are adjusted to improve the selected metric, changes to the identified primary parameters can be minimized.

Implementations could also have the clinician input what they want in clinical semantic language, with the system providing the best treatment plan with the best outcome. The Optimizer 218 can then provide to the clinician, through selected metric percentile scores, how well the predicted treatment plan compares to selected prior treatment plans. The Optimizer 218 can identify several treatment trade-offs, such that the suggested treatment alternatives will be those alternatives that are driving the selected parameters of the objective functions to improve the identified metrics the most. In one example, the clinician is notified that the tracked metrics for the predicted outcome exceed or meet the mean knowledge base result on every attribute. Such a result can be accepted and the Optimizer 218 will therefore finish and a final treatment plan will be created. Otherwise additional treatment alternatives can be selected to attempt to improve current attribute/metric percentile comparison scores.

### Further Optimization of Radiation Therapy Treatment Plans:

In a further embodiment, after the Optimizer 218 has completed optimizing the treatment plan results and a final treatment plan has been established, another clinician can request additional alternatives to further improve the treatment plan results. Furthermore, the current state of the treatment plan can be only a starting point. The optimization system can then go back to the knowledge base and after acquiring further statistical information from additional prior treatment plans, alternative treatment types, and improved statistical models, allow the optimizer to offer additional treatment alternatives that can be used to further improve the treatment plan result as defined in the metric percentile scores. In another embodiment, the system can go back to the knowledge base and look for prior treatment plans that might have been equally good, but have other attributes that might also be appealing to the clinician or the patient: such as time, expense, and other factors. Given all the objectives available, the system can find a universe of possible results that score the same on the objective functions with similar metric percentile scores, and allow the clinician to quickly navigate along the identified aggregate of results that can be applied, to find a preferred treatment plan and predicted result.

In another embodiment, the optimization can be reduced into component parts, such as a first series of highest objectives that have to be met as determined by the clinician and then the optimization system will later go back and try to improve all the other remaining objectives. The system could also offer the clinician with alternatives that will improve these other objectives.

In another embodiment, additional time can be spent in trying to meet the additional objectives. The clinician can determine how long the system is to work on optimizing the objectives. For example, after reaching an approved result, the optimization system can continue trying to improve the results for the remaining objectives and if improved results that exceed a predetermined threshold are identified, a selection of treatment alternatives can be offered to the clinician for further improvement of the treatment plan. Otherwise, the clinician approved treatment plan is used.

In another embodiment, there can be an optimization history. The system could approach the clinicians after the fact and review the parameters and decisions made in the treatment plan, such as: what the initial parameters were, what action was taken (to lower certain doses or raise a target dose, for example), or changes to make the treatment more robust. Such an optimization history could be implemented by having all those decisions mapped as human readable or understanding language.

Therefore, in an exemplary embodiment, human semantics are mapped to human understandable objectives that affect desired treatment plan attributes or metrics. Furthermore, the complex field of parameters, objectives, and metrics can be reduced into a smaller list of key drivers. The selected driving parameters can also be ranked such that once the primary parameters are established, the remaining parameters can be further adjusted such that while not affecting the primary parameters, adjusting the remaining parameters can affect their associated objective functions and result in improved attributes or metrics. Finally, the clinician can be provided, in clinically meaningful, human understandable language, what the treatment trade-offs are that the system is experiencing (e.g., that a particular parameter could be relaxed in return for a better result). A good trade-off can be determined through comparative analysis of prior treatment plans, such that a treatment alternative is a particular treatment trade-off between competing objectives, such that changing one objective to improve an attribute's or metric's percentile score will be at the expense of a competing objective.

The system will also capture in human semantic language those decisions that the clinician made that overrode or contradicted the recommended treatment plan provided by the knowledge base. The system will further ask the clinician why the change was made with the clinician able to provide a reason in clinically meaningful human understandable language. Such implementations could provide a semantic framework from which the clinician can select from to state their reasons (such as found in a drop-down menu of reasons). Otherwise, the clinician could also document in human understandable language, what they did and why. Finally the clinician approves the treatment plan. As discussed above, the optimization system 218 can also continue attempting to further optimize the current treatment plan by looking for further treatment alternatives that will improve the identified attributes or metrics by a threshold amount.

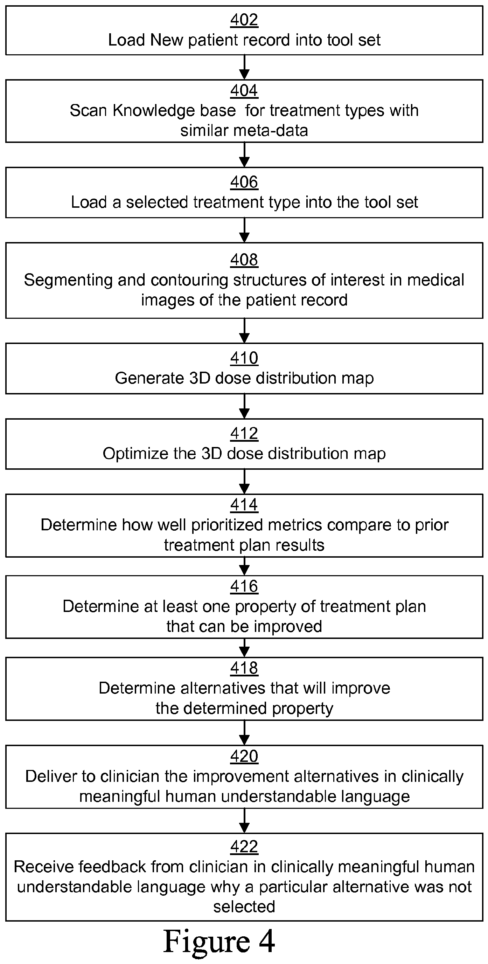

Figure 4 illustrates the steps to a method for optimizing a radiation therapy treatment plan with a prescribed radiation therapy dose distribution. In step 402, a new patient record 112 is loaded into the tool set 110. In step 404, the knowledge base 102 is scanned for treatment types 106 used by previous patient records 104 with similar metadata. In step 406, the selected treatment type 114 is downloaded into the tool set 110. In step 408, the medical image processing module 116 segments and contours the structures of interest (e.g., organs) in the medical images of the patient record 112.

In step 410, a 3D dose distribution map is generated based upon a downloaded average dose distance to target histogram. In step 412, based on field arrangements, a dose distribution in the patient record, selected and determined organ at risk objectives (e.g., average organ at risk dose volume histograms, cohort average organ at risk dose volume histograms, which are used to form 2D penalty maps), an optimized 3D dose distribution, associated fluences, and a dose volume histogram are generated by the Optimizer 218 and stored in the patient record. In step 414, the Optimizer 218 determines a percentile ranking for a series of identified priority metrics as compared to prior treatment plan results and the associated statistical models.

In step 416, the Optimizer 218 identifies at least one treatment alternative that results in an improved attribute or metric percentile score by adjusting the parameters of an objective to improve the attribute or metric at the expense of an opposing objective. Exemplary objectives can include at least one of: a more homogeneous target dose, less dose to critical target organs, to get a bigger difference between a target dose level and a critical organ dose level, lower MU's, and shorter treatment time, to name a few. In step 418, the Optimizer 218 detects possible objective trade-offs that will improve the desired attribute or metric. Example treatment alternatives can include at least one of: an increased number of fields, changing field geometry, increasing weights, and changing tradeoffs between conflicting objectives. In step 420, the Optimizer 218 offers the identified treatment alternatives to the clinician in clinically meaningful human understandable language. In step 422, the Optimizer 218 receives feedback from the clinician/user in clinically meaningful human understandable language, why a particular improvement alternative was not selected.

Although certain preferred embodiments and methods have been disclosed herein, it will be apparent from the foregoing disclosure to those skilled in the art that variations and modifications of such embodiments and methods may be made without departing from the scope of the invention. It is intended that the invention shall be limited only to the extent required by the appended claims.

## Claims

1. A computer-implemented method for radiation therapy treatment plan optimization based upon analysis of trade-offs between competing radiation therapy treatment plan objectives, wherein objectives relate to radiation therapy treatment plan goals, said method comprising:
loading a patient record including medical images into a treatment planning tool set (402); scanning a knowledge base for treatment types used by previous patient records with similar metadata (404), wherein the knowledge base includes previous treatment plans;
selecting and downloading a selected treatment type of the treatment types into the treatment planning tool set (406);
segmenting and contouring structures of interest in medical images of the patient record (408);
generating a three-dimensional dose distribution map (410) using the selected treatment type;
optimizing the three-dimensional dose distribution map (412);
generating a first radiation therapy treatment plan using the selected treatment type with the treatment planning tool set (302);
identifying a selection of metrics of the first radiation therapy treatment plan, wherein a metric of a radiation therapy treatment plan relates to how well the radiation therapy treatment plan performs in relation to an objective,
determining a percentile score for each of the metrics of the selection of metrics, said percentile scores defining how well the first radiation therapy treatment plan compares to previous treatment plans selected from the knowledge base (414);
identifying at least one parameter associated with a first objective of the first radiation treatment plan that affects an identified metric of the selection of metrics;
comparing the at least one parameter for the first objective to at least one parameter for a competing objective, identifying a possible trade-off that will improve the identified metric (418), wherein said trade-off is between competing objectives such that changing the at least one parameter of the first objective to improve the identified metric is at the expense of said competing objective; and
determining at least one alternative treatment step for the first radiation therapy treatment plan that will change the at least one parameter for the first objective to improve the percentile score of the identified metric at the expense of the competing objective.

2. The computer-implemented method of Claim 1 further comprising receiving feedback in human understandable language why at least one of the at least one alternative treatment step is not to be implemented in the first radiation therapy treatment plan, wherein the at least one alternative treatment step is expressed in human understandable language.

3. The computer-implemented method of Claim 1, wherein the first radiation therapy treatment plan is generated based upon patient metadata, the patient record, the selected treatment type, selected patient records of the previous patient records, and associated statistical models based on prior treatment plans approved by a clinician, and wherein the first radiation therapy treatment plan is optimized based upon the statistical models, selected objectives, and their associated parameters, wherein said parameters affect metrics of said selected objectives.

4. The computer-implemented method of Claim 1, wherein said percentile scores comprise a percentile score for each metric of the selection of metrics, wherein the percentile score for a metric defines a percentage of plans of other patient records, such that the percentile score for said metric is as high or higher than the scores achieved by said percentage of plans of other patient records; and wherein comparing the at least one parameter for the first objective to at least one parameter for the competing objective comprises analyzing the parameters that affect the identified metric with the at least one parameter for the competing objective that resulted in the trade-off, and wherein the changed at least one parameter for the first objective will improve the percentile score for a metric of higher priority while lowering the percentile score for another metric of lower priority, wherein metrics of higher priority are weighed higher than metrics of lower priority.

5. The computer-implemented method of Claim 4 further comprising reviewing the knowledge base for additional prior patient records and additional treatment types to generate additional alternative treatment steps that would result in improved percentile scores for the metrics of the first radiation therapy treatment plan that are above a defined improvement threshold, and wherein parameters that affect the metrics for the first radiation therapy treatment plan are ranked, such that once a setting for a higher ranking parameter has been established, settings for lower ranking parameters can be adjusted without changing the settings for the higher ranking parameter.

6. A computer readable storage media having computer-readable and computer-executable instructions embodied therein for causing a computer system to execute a method for radiation therapy treatment plan optimization based upon analysis of trade-offs between competing radiation therapy treatment plan objectives, wherein objectives relate to radiation therapy treatment plan goals, the computer-executable instructions comprising:
instructions to load a patient record including medical images into a treatment planning tool set;
instructions to scan a knowledge base for treatment types used by previous patient records with similar metadata, wherein the knowledge base includes previous treatment plans;
instructions to select a treatment type of the treatment types;instructions to download the selected treatment type into the treatment planning tool set;
instructions to segment and contour structures of interest in medical images of the patient record;
instructions to generate a three-dimensional dose distribution map using the selected treatment type;
instructions to optimize the three-dimensional dose distribution map;
instructions to generate a first radiation therapy treatment plan using the selected treatment type with the treatment planning tool set;
instructions to identify a selection of metrics of the first radiation therapy treatment plan, wherein a metric of a radiation therapy treatment plan relates to how well the radiation therapy treatment plan performs in relation to an objective;
instructions to determine a percentile score for each metric of the selection of metrics of the first radiation therapy treatment plan, said percentile scores defining how well the first radiation therapy treatment plan compares to previous treatment plans selected from the knowledge base;
instructions to identify at least one parameter associated with a first objective of the first radiation treatment plan that affects an identified metric of the selection of metrics;
instructions to compare the at least one parameter for the first objective to at least one parameter for a competing objective to identify a possible trade-off that will improve the identified metric, wherein said trade-off is between competing objectives such that changing the at least one parameter of the first objective to improve the identified metric is at the expense of said competing objective;
instructions to determine at least one alternative treatment step for the first radiation therapy treatment plan that will change the at least one parameter for the first objective to improve the percentile score of the identified metric at the expense of the competing objective;
instructions to offer the at least one alternative treatment step to a user in human understandable language; and
instructions to receive feedback from the user, in human understandable language, a reason that an alternative treatment step of the at least one alternative treatment step is not to be implemented.

7. The computer readable storage media of Claim 6, wherein the instructions to generate the first radiation therapy treatment plan are based upon patient metadata, the patient record, selected treatment type, selected patient record of the previous patient records, and associated statistical models based on prior treatment plans approved by a clinician, and wherein the instructions to optimize the first radiation therapy treatment plan are based upon the statistical models, selected objectives and their associated parameters, wherein said parameters affect metrics of said selected objectives.

8. The computer readable storage media of Claim 6, wherein said percentile scores comprise a percentile score for each metric of the selection of metrics, wherein the percentile score for a metric defines a percentage of plans of other patient records, such that the score for said metric is as high or higher than the scores achieved by said percentage of plans of other patient records.

9. The computer readable storage media of Claim 6, wherein the instructions to compare the at least one parameter for the first objective to at least one parameter for the competing objective comprise analyzing the parameters that affect the identified metric with the at least one parameter for the competing metric that resulted in the trade-off, wherein the at least one parameter for the first objective will improve the percentile score for a metric of higher priority while lowering the percentile score for another metric of lower priority, wherein metrics of higher priority are weighed higher than metrics of lower priority; and wherein parameters that affect the metrics for the first radiation therapy treatment plan are ranked, such that once a setting for a higher ranking parameter has been established, settings for lower ranking parameters can be adjusted without changing the setting for the higher ranking parameter.

10. The computer readable storage media of Claim 6, wherein the computer-executable instructions further comprise instructions to review the knowledge base for additional prior patient records and additional treatment types to generate additional alternative treatment steps that would result in improved metrics for the first radiation therapy treatment plan that are above a defined improvement threshold.

## Patentansprüche

1. Computerimplementiertes Verfahren zur Optimierung eines Bestrahlungstherapiebehandlungsplans auf Grundlage einer Analyse von Trade-offs zwischen konkurrierenden Zielsetzungen eines Bestrahlungstherapiebehandlungsplans, wobei Zielsetzungen Ziele von Bestrahlungstherapiebehandlungsplänen betreffen, wobei das Verfahren Folgendes umfasst:
Laden einer Patientenakte, einschließend medizinische Bilder, in einen Werkzeugsatz für die Behandlungsplanung (402);
Durchsuchen einer Wissensdatenbank im Hinblick auf in früheren Patientenakten angewendete Behandlungstypen mit ähnlichen Metadaten (404), wobei die Wissensdatenbank frühere Behandlungspläne einschließt;
Auswählen und Herunterladen eines ausgewählten Behandlungstyps der Behandlungstypen in den Werkzeugsatz für die Behandlungsplanung (406);
Segmentieren und Konturieren von interessanten Strukturen in medizinischen Bildern der Patientenakte (408);
Generieren einer dreidimensionalen Dosisverteilungskarte (410) unter Verwendung des ausgewählten Behandlungstyps;
Optimieren der dreidimensionalen Dosisverteilungskarte (412);
Generieren eines ersten Bestrahlungstherapiebehandlungsplans unter Verwendung des ausgewählten Behandlungstyps mit dem Werkzeugsatz für die Behandlungsplanung (302);
Identifizieren einer Auswahl von Metriken des ersten Bestrahlungstherapiebehandlungsplans, wobei eine Metrik eines Bestrahlungstherapiebehandlungsplans betrifft, wie gut der Bestrahlungstherapiebehandlungsplan in Bezug auf eine Zielsetzung abschneidet,
Bestimmen eines Perzentilwerts für jede der Metriken der Auswahl von Metriken, wobei die Perzentilwerte definieren, wie gut der erste Bestrahlungstherapiebehandlungsplan im Vergleich mit früheren Behandlungsplänen dasteht, die aus der Wissensdatenbank ausgewählt werden (414);
Identifizieren mindestens eines Parameters, der mit einer ersten Zielsetzung des ersten Bestrahlungsbehandlungsplans verknüpft ist, der eine identifizierte Metrik der Auswahl von Metriken beeinflusst;
Vergleichen des mindestens einen Parameters für die erste Zielsetzung mit mindestens einem Parameter für eine konkurrierende Zielsetzung, wobei ein möglicher Trade-off identifiziert wird, der die identifizierte Metrik verbessert (418), wobei der Trade-off zwischen konkurrierenden Zielsetzungen dergestalt ist, dass eine Änderung des mindestens einen Parameters der ersten Zielsetzung zum Verbessern der identifizierten Metrik auf Kosten der konkurrierenden Zielsetzung geht; und
Bestimmen von mindestens einem alternativen Behandlungsschritt für den ersten Bestrahlungstherapiebehandlungsplan, der den mindestens einen Parameter für die erste Zielsetzung ändert, um den Perzentilwert der identifizierten Metrik auf Kosten der konkurrierenden Zielsetzung zu verbessern.

2. Computerimplementiertes Verfahren nach Anspruch 1, ferner umfassend das Empfangen einer Rückmeldung in für den Menschen verständlicher Sprache dafür, warum mindestens einer des mindestens einen alternativen Behandlungsschritts nicht in dem ersten Bestrahlungstherapiebehandlungsplan umgesetzt werden soll, wobei der mindestens eine alternative Behandlungsschritt in für den Menschen verständlicher Sprache ausgedrückt wird.

3. Computerimplementiertes Verfahren nach Anspruch 1, wobei der erste Bestrahlungstherapiebehandlungsplan auf Grundlage von Patientenmetadaten, der Patientenakte, dem ausgewählten Behandlungstyp, ausgewählten Patientenakten der früheren Patientenakten und zugehörigen statistischen Modellen auf Grundlage von vorausgehenden Behandlungsplänen generiert wird, die von einem Klinikarzt genehmigt wurden, und wobei der erste Bestrahlungstherapiebehandlungsplan auf Grundlage der statistischen Modelle, ausgewählter Zielsetzungen und ihrer zugehörigen Parameter optimiert wird, wobei die Parameter Metriken der ausgewählten Zielsetzungen beeinflussen.

4. Computerimplementiertes Verfahren nach Anspruch 1, wobei die Perzentilwerte einen Perzentilwert für jede Metrik der Auswahl von Metriken umfassen, wobei der Perzentilwert für eine Metrik einen Prozentsatz von Plänen aus anderen Patientenakten definiert, sodass der Perzentilwert für die Metrik so hoch ist wie die Werte, die durch den Prozentsatz von Plänen aus anderen Patientenakten erreicht werden oder höher; und wobei das Vergleichen des mindestens einen Parameters für die erste Zielsetzung mit mindestens einem Parameter für die konkurrierende Zielsetzung das Analysieren der Parameter, welche die identifizierte Metrik beeinflussen, mit dem mindestens einen Parameter für die konkurrierende Zielsetzung umfasst, der zum Trade-off geführt hat, und wobei der geänderte mindestens eine Parameter für die erste Zielsetzung den Perzentilwert für eine Metrik mit einer höheren Priorität verbessert, während er den Perzentilwert für eine andere Metrik mit niedrigerer Priorität senkt, wobei Metriken mit einer höheren Priorität höher gewichtet werden als Metriken mit niedrigerer Priorität.

5. Computerimplementiertes Verfahren nach Anspruch 4, ferner umfassend das Überprüfen der Wissensdatenbank im Hinblick auf zusätzliche vorausgehende Patientenakten und zusätzliche Behandlungstypen, um zusätzliche alternative Behandlungsschritte zu generieren, die verbesserte Perzentilwerte für die Metriken des ersten Bestrahlungstherapiebehandlungsplans ergeben, die über einer definierten Verbesserungsschwelle liegen, und wobei Parameter, welche die Metriken für den ersten Bestrahlungstherapiebehandlungsplan beeinflussen, eingestuft werden, sodass, sobald eine Einstellung für einen Parameter mit höherer Einstufung festgelegt wurde, Einstellungen für Parameter mit einer niedrigeren Einstufung angepasst werden können, ohne die Einstellungen für den Parameter mit der höheren Einstufung zu ändern.

6. Computerlesbare Speichermedien, die computerlesbare und von einem Computer ausführbare Anweisungen aufweisen, die darin ausgeführt sind, um zu veranlassen, dass ein Computersystem ein Verfahren zur Optimierung eines Bestrahlungstherapiebehandlungsplans auf Grundlage einer Analyse von Trade-offs zwischen konkurrierenden Zielsetzungen eines Bestrahlungstherapiebehandlungsplans ausführt, wobei Zielsetzungen Ziele von Bestrahlungstherapiebehandlungsplänen betreffen, wobei die von einem Computer ausführbaren Anweisungen Folgendes umfassen:
Anweisungen zum Laden einer Patientenakte, einschließend medizinische Bilder, in einen Werkzeugsatz für die Behandlungsplanung;
Anweisungen zum Durchsuchen einer Wissensdatenbank im Hinblick auf in früheren Patientenakten angewendete Behandlungstypen mit ähnlichen Metadaten, wobei die Wissensdatenbank frühere Behandlungspläne einschließt;
Anweisungen zum Auswählen eines Behandlungstyps der Behandlungstypen; Anweisungen zum Herunterladen des ausgewählten Behandlungstyps in den Werkzeugsatz für die Behandlungsplanung;
Anweisungen zum Segmentieren und Konturieren von interessanten Strukturen in medizinischen Bildern der Patientenakte;
Anweisungen zum Generieren einer dreidimensionalen Dosisverteilungskarte unter Verwendung des ausgewählten Behandlungstyps;
Anweisungen zum Optimieren der dreidimensionalen Dosisverteilungskarte;
Anweisungen zum Generieren eines ersten Bestrahlungstherapiebehandlungsplans unter Verwendung des ausgewählten Behandlungstyps mit dem Werkzeugsatz für die Behandlungsplanung;
Anweisungen zum Identifizieren einer Auswahl von Metriken des ersten Bestrahlungstherapiebehandlungsplans, wobei eine Metrik eines Bestrahlungstherapiebehandlungsplans betrifft, wie gut der Bestrahlungstherapiebehandlungsplan in Bezug auf eine Zielsetzung abschneidet;
Anweisungen zum Bestimmen eines Perzentilwerts für jede Metrik der Auswahl von Metriken des ersten Bestrahlungstherapiebehandlungsplans, wobei die Perzentilwerte definieren, wie gut der erste Bestrahlungstherapiebehandlungsplan im Vergleich mit früheren Behandlungsplänen dasteht, die aus der Wissensdatenbank ausgewählt werden;
Anweisungen zum Identifizieren mindestens eines Parameters, der mit einer ersten Zielsetzung des ersten Bestrahlungsbehandlungsplans verknüpft ist, der eine identifizierte Metrik der Auswahl von Metriken beeinflusst;
Anweisungen zum Vergleichen des mindestens einen Parameters für die erste Zielsetzung mit mindestens einem Parameter für eine konkurrierende Zielsetzung, um einen möglichen Trade-off zu identifizieren, der die identifizierte Metrik verbessert, wobei der Trade-off zwischen konkurrierenden Zielsetzungen dergestalt ist, dass eine Änderung des mindestens einen Parameters der ersten Zielsetzung zum Verbessern der identifizierten Metrik auf Kosten der konkurrierenden Zielsetzung geht;
Anweisungen zum Bestimmen von mindestens einem alternativen Behandlungsschritt für den ersten Bestrahlungstherapiebehandlungsplan, der den mindestens einen Parameter für die erste Zielsetzung ändert, um den Perzentilwert der identifizierten Metrik auf Kosten der konkurrierenden Zielsetzung zu verbessern;
Anweisungen zum Anbieten des mindestens einen alternativen Behandlungsschritts für einen Benutzer in für den Menschen verständlicher Sprache; und
Anweisungen zum Empfangen einer Rückmeldung von dem Benutzer in für den Menschen verständlicher Sprache mit einer Begründung dafür, dass ein alternativer Behandlungsschritt des mindestens einen alternativen Behandlungsschritts nicht umgesetzt werden soll.

7. Computerlesbare Speichermedien nach Anspruch 6, wobei die Anweisungen zum Generieren des ersten Bestrahlungstherapiebehandlungsplans auf Patientenmetadaten, der Patientenakte, einem ausgewählten Behandlungstyp, einer ausgewählten Patientenakte der früheren Patientenakten und zugehörigen statistischen Modellen auf Grundlage von vorausgehenden Behandlungsplänen basieren, die von einem Klinikarzt genehmigt wurden, und wobei die Anweisungen zum Optimieren des ersten Bestrahlungstherapiebehandlungsplans auf den statistischen Modellen, ausgewählten Zielsetzungen und ihren zugehörigen Parametern basieren, wobei die Parameter Metriken der ausgewählten Zielsetzungen beeinflussen.

8. Computerlesbare Speichermedien nach Anspruch 6, wobei die Perzentilwerte einen Perzentilwert für jede Metrik der Auswahl von Metriken umfassen, wobei der Perzentilwert für eine Metrik einen Prozentsatz von Plänen aus anderen Patientenakten definiert, sodass der Wert für die Metrik so hoch ist wie die Werte, die durch den Prozentsatz von Plänen aus anderen Patientenakten erreicht werden oder höher.

9. Computerlesbare Speichermedien nach Anspruch 6, wobei die Anweisungen zum Vergleichen des mindestens einen Parameters für die erste Zielsetzung mit mindestens einem Parameter für die konkurrierende Zielsetzung das Analysieren der Parameter, welche die identifizierte Metrik beeinflussen, mit dem mindestens einen Parameter für die konkurrierende Metrik umfassen, der zum Trade-off geführt hat, wobei der mindestens eine Parameter für die erste Zielsetzung den Perzentilwert für eine Metrik mit einer höheren Priorität verbessert, während er den Perzentilwert für eine andere Metrik mit niedrigerer Priorität senkt, wobei Metriken mit einer höheren Priorität höher gewichtet werden als Metriken mit niedrigerer Priorität; und wobei Parameter, welche die Metriken für den ersten Bestrahlungstherapiebehandlungsplan beeinflussen, eingestuft werden, sodass, sobald eine Einstellung für einen Parameter mit höherer Einstufung festgelegt wurde, Einstellungen für Parameter mit einer niedrigeren Einstufung angepasst werden können, ohne die Einstellung für den Parameter mit der höheren Einstufung zu ändern.

10. Computerlesbare Speichermedien nach Anspruch 6, wobei die von einem Computer ausführbaren Anweisungen ferner Anweisungen zum Überprüfen der Wissensdatenbank im Hinblick auf zusätzliche vorausgehende Patientenakten und zusätzliche Behandlungstypen umfassen, um zusätzliche alternative Behandlungsschritte zu generieren, die verbesserte Metriken für den ersten Bestrahlungstherapiebehandlungsplan ergeben, die über einer definierten Verbesserungsschwelle liegen.

## Revendications

1. Procédé mis en œuvre par ordinateur pour l'optimisation de plan de traitement de radiothérapie sur la base de l'analyse de compromis entre des objectifs de plan de traitement de radiothérapie concurrents, dans lequel les objectifs se rapportent aux buts de plan de traitement de radiothérapie, ledit procédé comprenant :
le chargement d'un dossier de patient comportant des images médicales dans un ensemble d'outils de planification de traitement (402) ;
le balayage d'une base de connaissances pour des types de traitement utilisés par des dossiers de patient antérieurs avec des métadonnées similaires (404), dans lequel la base de connaissances comporte des plans de traitement antérieurs ;
la sélection et le téléchargement d'un type de traitement sélectionné parmi les types de traitement dans l'ensemble d'outils de planification de traitement (406) ;
la segmentation et le contournage de structures d'intérêt dans des images médicales du dossier de patient (408) ;
la génération d'une carte de distribution de dose tridimensionnelle (410) à l'aide du type de traitement sélectionné ;
l'optimisation de la carte de distribution de dose tridimensionnelle (412) ;
la génération d'un premier plan de traitement de radiothérapie à l'aide du type de traitement sélectionné avec l'ensemble d'outils de planification de traitement (302) ;
l'identification d'une sélection de métriques du premier plan de traitement de radiothérapie, dans lequel une métrique d'un plan de traitement de radiothérapie se rapporte à la mesure dans laquelle le plan de traitement de radiothérapie fonctionne par rapport à un objectif,
la détermination d'un score de centile pour chacune des métriques de la sélection de métriques, lesdits scores de centile définissant dans quelle mesure le premier plan de traitement de radiothérapie se compare aux plans de traitement antérieurs sélectionnés à partir de la base de connaissances (414) ;
l'identification d'au moins un paramètre associé à un premier objectif du premier plan de traitement de radiothérapie qui affecte une métrique identifiée de la sélection de métriques ;
la comparaison de l'au moins un paramètre pour le premier objectif à au moins un paramètre pour un objectif concurrent, l'identification d'un compromis possible qui améliorera la métrique identifiée (418), dans lequel ledit compromis est entre des objectifs concurrents de sorte que le changement de l'au moins un paramètre du premier objectif pour améliorer la métrique identifiée se fait aux dépens dudit objectif concurrent ; et
la détermination d'au moins une étape de traitement alternative pour le premier plan de traitement de radiothérapie qui modifiera l'au moins un paramètre pour le premier objectif afin d'améliorer le score de centile de la métrique identifiée aux dépens de l'objectif concurrent.

2. Procédé mis en œuvre par ordinateur selon la revendication 1, comprenant en outre la réception d'une rétroaction dans un langage compréhensible par l'homme expliquant pourquoi au moins l'une de l'au moins une étape de traitement alternative ne doit pas être mise en œuvre dans le premier plan de traitement de radiothérapie, dans lequel l'au moins une étape de traitement alternative est exprimée dans un langage compréhensible par l'homme.

3. Procédé mis en œuvre par ordinateur selon la revendication 1, dans lequel le premier plan de traitement de radiothérapie est généré sur la base de métadonnées de patient, du dossier de patient, du type de traitement sélectionné, des dossiers de patient sélectionnés parmi les dossiers de patient antérieurs et des modèles statistiques associés sur la base des plans de traitement antérieurs approuvés par un clinicien, et dans lequel le premier plan de traitement de radiothérapie est optimisé sur la base des modèles statistiques, des objectifs sélectionnés et de leurs paramètres associés, dans lequel lesdits paramètres affectent les métriques desdits objectifs sélectionnés.

4. Procédé mis en œuvre par ordinateur selon la revendication 1, dans lequel lesdits scores de centile comprennent un score de centile pour chaque métrique de la sélection de métriques, dans lequel le score de centile pour une métrique définit un pourcentage de plans d'autres dossiers de patient, de sorte que le score de centile pour ladite métrique est aussi élevé ou plus élevé que les scores obtenus par ledit pourcentage de plans d'autres dossiers de patient ; et dans lequel la comparaison de l'au moins un paramètre pour le premier objectif à au moins un paramètre pour l'objectif concurrent comprend l'analyse des paramètres qui affectent la métrique identifiée avec l'au moins un paramètre pour l'objectif concurrent qui a entraîné le compromis, et dans lequel l'au moins un paramètre modifié pour le premier objectif améliorera le score de centile pour une métrique de priorité supérieure tout en baissant le score de centile pour une autre métrique de priorité inférieure, dans lequel les métriques de priorité supérieure ont une pondération plus élevée que les métriques de priorité inférieure.

5. Procédé mis en œuvre par ordinateur selon la revendication 4, comprenant en outre l'examen de la base de connaissances pour des dossiers de patient antérieurs supplémentaires et des types de traitement supplémentaires afin de générer des étapes de traitement alternatives supplémentaires qui entraîneraient des scores de centile améliorés pour les métriques du premier plan de traitement de radiothérapie qui sont supérieurs à un seuil d'amélioration défini, et dans lequel les paramètres qui affectent les métriques pour le premier plan de traitement de radiothérapie sont classés, de sorte qu'une fois qu'un réglage pour un paramètre de classement supérieur a été établi, les réglages pour les paramètres de classement inférieur peuvent être ajustés sans modifier les réglages pour le paramètre de classement supérieur.

6. Support de stockage lisible par ordinateur comportant des instructions lisibles par ordinateur et exécutables par ordinateur pour amener un système informatique à exécuter un procédé d'optimisation de plan de traitement de radiothérapie sur la base de l'analyse de compromis entre des objectifs de plan de traitement de radiothérapie concurrents, dans lequel les objectifs se rapportent à des buts de plan de traitement de radiothérapie, les instructions exécutables par ordinateur comprenant :
des instructions pour charger un dossier de patient comportant des images médicales dans un ensemble d'outils de planification de traitement ;
des instructions pour numériser une base de connaissances pour des types de traitement utilisés par des dossiers de patient antérieurs avec des métadonnées similaires, dans lequel la base de connaissances comporte des plans de traitement antérieurs ;
des instructions pour sélectionner un type de traitement parmi les types de traitement ;
des instructions pour télécharger le type de traitement sélectionné dans l'ensemble d'outils de planification de traitement ;
des instructions pour segmenter et contourner des structures d'intérêt dans les images médicales du dossier de patient ;
des instructions pour générer une carte de distribution de dose tridimensionnelle à l'aide du type de traitement sélectionné ;
des instructions pour optimiser la carte de distribution de dose tridimensionnelle ;
des instructions pour générer un premier plan de traitement de radiothérapie à l'aide du type de traitement sélectionné avec l'ensemble d'outils de planification de traitement ;
des instructions pour identifier une sélection de métriques du premier plan de traitement de radiothérapie, dans lequel une métrique d'un plan de traitement de radiothérapie se rapporte à la mesure dans laquelle le plan de traitement de radiothérapie fonctionne par rapport à un objectif ;
des instructions pour déterminer un score de centile pour chaque métrique de la sélection de métriques du premier plan de traitement de radiothérapie, lesdits scores de centile définissant dans quelle mesure le premier plan de traitement de radiothérapie se compare aux plans de traitement antérieurs sélectionnés à partir de la base de connaissances ;
des instructions pour identifier au moins un paramètre associé à un premier objectif du premier plan de radiothérapie qui affecte une métrique identifiée de la sélection de métriques ;
des instructions pour comparer l'au moins un paramètre pour le premier objectif à au moins un paramètre pour un objectif concurrent pour identifier un compromis possible qui améliorera la métrique identifiée, dans lequel ledit compromis est entre des objectifs concurrents de sorte que le changement de l'au moins un paramètre du premier objectif pour améliorer la métrique identifiée se fait aux dépens dudit objectif concurrent ;
des instructions pour déterminer au moins une étape de traitement alternative pour le premier plan de traitement de radiothérapie qui modifiera l'au moins un paramètre pour le premier objectif afin d'améliorer le score de centile de la métrique identifiée aux dépens de l'objectif concurrent ;
des instructions pour proposer l'au moins une étape de traitement alternative à un utilisateur dans un langage compréhensible par l'homme ; et
des instructions pour recevoir une rétroaction de l'utilisateur, dans un langage compréhensible par l'homme, une raison pour laquelle une étape de traitement alternative de l'au moins une étape de traitement alternative ne doit pas être mise en œuvre.

7. Support de stockage lisible par ordinateur selon la revendication 6, dans lequel les instructions pour générer le premier plan de traitement de radiothérapie sont basées sur les métadonnées de patient, le dossier de patient, le type de traitement sélectionné, le dossier de patient sélectionné parmi les dossiers de patient antérieurs, et les modèles statistiques associés sur la base des plans de traitement antérieurs approuvés par un clinicien, et dans lequel les instructions pour optimiser le premier plan de traitement de radiothérapie sont basées sur les modèles statistiques, les objectifs sélectionnés et leurs paramètres associés, dans lequel lesdits paramètres affectent les métriques desdits objectifs sélectionnés.

8. Support de stockage lisible par ordinateur selon la revendication 6, dans lequel lesdits scores de centile comprennent un score de centile pour chaque métrique de la sélection de métriques, dans lequel le score de centile pour une métrique définit un pourcentage de plans d'autres dossiers de patient, de sorte que le score pour ladite métrique est aussi élevé ou plus élevé que les scores obtenus par ledit pourcentage de plans d'autres dossiers de patient.

9. Support de stockage lisible par ordinateur selon la revendication 6, dans lequel les instructions pour comparer l'au moins un paramètre pour le premier objectif à au moins un paramètre pour l'objectif concurrent comprennent l'analyse des paramètres qui affectent la métrique identifiée avec l'au moins un paramètre pour la métrique concurrente qui a abouti au compromis, dans lequel l'au moins un paramètre pour le premier objectif améliorera le score de centile pour une métrique de priorité plus élevée tout en baissant le score de centile pour une autre métrique de priorité inférieure, dans lequel les métriques de priorité supérieure ont une pondération plus élevée que les métriques de priorité inférieure ; et dans lequel les paramètres qui affectent les métriques pour le premier plan de traitement de radiothérapie sont classés, de sorte qu'une fois qu'un réglage pour un paramètre de rang supérieur a été établi, les réglages pour des paramètres de rang inférieur peuvent être ajustés sans modifier le réglage pour le paramètre de rang supérieur.

10. Support de stockage lisible par ordinateur selon la revendication 6, dans lequel les instructions exécutables par ordinateur comprennent en outre des instructions pour examiner la base de connaissances pour des dossiers de patient antérieurs supplémentaires et des types de traitement supplémentaires afin de générer des étapes de traitement alternatives supplémentaires qui entraîneraient des métriques améliorées pour le premier plan de traitement de radiothérapie qui sont supérieures à un seuil d'amélioration défini.
